# EUROPÄISCHE PATENTSCHRIFT

(11) **EP 1 834 263 B1**
(45) Veröffentlichungstag und Bekanntmachung des Hinweises auf die Patenterteilung: **06.10.2010**
(21) Anmeldenummer: 05818436.7
(22) Anmeldetag: 20.12.2005
(51) Int. Cl.: G06F 19/00, G01N 33/487

(54) **MESSSYSTEM ZUR MESSUNG VON STOFFKONZENTRATIONEN IN FLUIDEN MEDIEN**
MEASURING SYSTEM FOR THE MEASUREMENT OF MATERIAL CONCENTRATIONS IN LIQUID MEDIA
SYSTEME DE MESURE SERVANT A MESURER DES CONCENTRATIONS DE SUBSTANCES DANS DES MILIEUX LIQUIDES

(30) Priorität: 23.12.2004 DE 102004062255
(43) Veröffentlichungstag der Anmeldung: 19.09.2007
(73) Patentinhaber: Roche Diagnostics GmbH, 68305 Mannheim (DE); F. Hoffmann-La Roche AG, 4070 Basel (CH)
(72) Erfinder: WOHLAND, Albert, 68519 Viernheim (DE)
(74) Vertreter: Stößel, Matthias
(86) Internationale Anmeldenummer: PCT/EP2005/013701
(87) Internationale Veröffentlichungsnummer: WO 2006/069675

(56) Entgegenhaltungen:
- EP-A- 1 225 448
- EP-B- 0 101 812
- WO-A-02/078533
- WO-A-03/082091
- WO-A-2004/090503
- DE-A1- 10 237 602
- US-A- 5 307 263

## Beschreibung

### Gebiet der Erfindung

Die Erfindung betrifft ein tragbares analytisches Messsystem zur Messung von Stoffkonzentrationen in fluiden Medien, insbesondere ein Messsystem zum Messen von Blutglukosekonzentrationen und/oder Blutfettkonzentrationen. Weiterhin betrifft die Erfindung ein Verfahren zur Messung von Stoffkonzentrationen in fluiden Medien mittels eines erfindungsgemäßen Messsystems sowie die Verwendung eines "intelligenten" Teststreifenbehältnisses in einem derartigen Messsystem zur Messung von Stoffkonzentrationen in fluiden Medien.

### Stand der Technik

Die Überwachung der Blutglukosekonzentration ist für Diabetiker ein essentieller Bestandteil des Tagesablaufs. Dabei muss die Blutglukosekonzentration schnell und einfach mehrmals am Tag bestimmt werden, um gegebenenfalls entsprechende medizinische Maßnahmen ergreifen zu können. Um den Tagesablauf des Diabetikers nicht mehr als nötig einzuschränken, werden dabei häufig entsprechende mobile Geräte eingesetzt, welche einfach zu transportieren und zu handhaben sein sollten, so dass die Messung der Blutglukosekonzentration beispielsweise am Arbeitsplatz oder auch in der Freizeit erfolgen kann.

Derzeit sind verschiedene mobile Geräte auf dem Markt, welche teilweise nach unterschiedlichen Messmethoden funktionieren. Dabei kommen verschiedene Diagnoseverfahren zum Einsatz, beispielsweise optische oder auch elektrochemische Messverfahren. Ein Beispiel eines häufig eingesetzten Messverfahrens nutzt eine spezielle Art elektrochemischer Teststreifen. Diese Teststreifen sind beispielsweise so aufgebaut, dass eine vorgegebene Blutmenge über ein Kapillarensystem in dem Teststreifen zu einem Elektrodensystem geführt wird. Für moderne Teststreifen genügt dabei eine Blutmenge von ca. 1,5 µl, teilweise auch Blutmengen unter 1 µl. Bei dem Elektrodensystem kann es sich zum Beispiel um Goldelektroden handeln, welche mit einer Beschichtung versehen sind. Die Beschichtung enthält zumeist verschiedene Enzyme und sogenannte Mediatoren und bewirkt, dass sich innerhalb der Probe an den Elektroden Ladungsträger (beispielsweise in Form von Redox-Molekülen) bilden, deren Konzentration abhängig ist von der Blutglukosekonzentration. Die Konzentration dieser Ladungsträger kann mittels der Goldelektroden und einem geeigneten Messsystem, beispielsweise mittels einer Strom-Spannungs-Messung, bestimmt werden, so dass daraus schließlich auf die Blutglukosekonzentration zurückgerechnet werden kann.

Ein wesentliches Element derartiger portabler Diagnosesysteme bilden somit die entsprechenden Teststreifen. Typischerweise werden von einem Diabetiker ca. 5 bis 7 derartiger Teststreifen pro Tag benötigt. Essentiell ist dabei, dass die Teststreifen sauber und trocken aufbewahrt werden, um nicht durch eine entsprechende Verschmutzung beziehungsweise Einwirkung von Feuchtigkeit die Messung der Blutglukosekonzentration zu verfälschen.

Häufig kommt es jedoch vor, dass der Nutzer beziehungsweise Patient ein Teststreifenbehältnis für Teststreifen nach der Entnahme eines oder mehrerer Teststreifen nicht korrekt verschließt. Auch kann es vorkommen, dass das Teststreifenbehältnis über längere Zeit überhaupt nicht geschlossen wird. Durch die dadurch verursachte Undichtigkeit beziehungsweise Lufteinwirkung auf die Teststreifen kann es zu Qualitätsbeeinträchtigungen der Teststreifen und zu falschen Messergebnissen kommen.

Weiterhin tritt bei den Teststreifen das Problem auf, dass die Eigenschaften der eingesetzten Teststreifen häufig von Charge zu Charge variieren können. Hierdurch ergeben sich teilweise massive Schwankungen der Genauigkeit der Blutglukosekonzentrationsmessung. Bei vielen bekannten kommerziellen Systemen müssen daher Chargeninformationen über die Teststreifen vor einer entsprechenden Blutglukosekonzentrationsmessung in ein Analysemodul eingegeben werden, damit das Analysemodul mittels dieser Chargeninformationen die Messdaten des Teststreifens richtig "interpretieren" kann, um daraus eine korrekte Blutglukosekonzentration zu ermitteln. Für diese Eingabe der Chargeninformationen in das Analysemodul existieren mehrere bekannte Verfahren. Beispielsweise kann hier mit entsprechenden Strichcodes gearbeitet werden, welche zum Beispiel auf dem Teststreifenbehältnis angebracht sind und welche vom Analysemodul ausgelesen werden können. Alternativ sind auch Systeme bekannt, bei denen dem Teststreifenbehältnis der Teststreifen ein Datenträger, häufig auch "ROM-Key" genannt, beigefügt wird. Dieser Datenträger kann beispielsweise lose dem verschlossenen Teststreifenbehältnis der Teststreifen beigefügt sein oder auch fest in das Teststreifenbehältnis integriert sein. Geht dieser ROM-Key jedoch (beispielsweise aufgrund einer Lösung vom Teststreifenbehältnis) verloren, so gehen mit ihm auch die Chargeninformationen verloren, und die Teststreifen sind somit weitgehend unbrauchbar.

Weiterhin haben die bekannten Arten der Übertragung von Chargeninformationen in das Analysemodul den Nachteil, dass jeweils eine entsprechende Aktion des Nutzers beziehungsweise Patienten vorausgesetzt wird. Eine automatische Übertragung der Chargeninformationen erfolgt nicht. Vergisst der Nutzer beziehungsweise der Patient jedoch, diese Chargeninformationen zu übertragen, oder tritt beim Übertragen der Chargeninformationen ein Fehler auf, so kann dies die fatale Folge haben, dass beispielsweise Blutglukosekonzentrationen falsch berechnet werden. Hierdurch können insbesondere falsche medizinische Gegenmaßnahmen, beispielsweise in Form falscher Dosierungen von Insulin, angeregt werden, was fatale Folgen für den Patienten haben kann.

Aus der EP 0 101 812B1 ist eine Vorrichtung zur Darreichung von Medikamenten bekannt, bei der ein Medikament von einem Behälter ausgegeben wird, welcher eine Kappe umfasst, wobei eine Sensoreinrichtung registriert, wenn die Kappe des Behälters geöffnet wird. Weiterhin umfasst der Behälter eine Uhr, welche die Zeitpunkte eines Öffnens beziehungsweise Schließens der Kappe registriert. Weiterhin sind eine Vorrichtung und eine Anzeige vorgesehen, über welche ein Patient darauf aufmerksam gemacht wird, wann ein Medikament wieder eingenommen werden muss.

Aus dem Bereich der Pharmazie sind mehrere Systeme bekannt, bei denen mittels sogenannter radio frequency identification (RFID) tags pharmazeutische Produkte verfolgt werden. Ein derartiges System ist beispielsweise aus der WO 03/071943 bekannt. In der dort beschriebenen Anordnung werden medizinische Produkte mit RFID tags versehen und in einem entsprechenden Speicherschrank aufbewahrt. Ein Empfangsgerät, welches mit einem entsprechenden Prozessor gekoppelt ist, empfängt die Signale der RFID tags der medizinische Produkte und kann beispielsweise automatisch eine Lagerverwaltung dieser Produkte organisieren.

WO 03/082091 offenbart ein tragbares analytisches Messystem zur Messung von Stoffkonzentrationen in fluiden Medien mit a) einem Teststreifenbehältnis zur Aufnahme mindestens eines Teststreifens, wobei das Teststreifenbehältnis folgendes aufweist eine Verschlusseinrichtung, eine Zählvorrichtung, einen Datenträger und b) einem

Analysemodul, wobei das Analysemodul folgendes aufweist eine Vorrichtung zum messen von Stoffkonzentration in fluiden Medien mittels eines Teststreifens.

### Aufgabe der Erfindung

Aufgabe der vorliegenden Erfindung ist es, ein System bereitzustellen, welches eine einfache und sichere Messung von Stoffkonzentrationen in fluiden Medien mittels Teststreifen ermöglicht. Das beschriebene System soll insbesondere die dargestellten Nachteile des Standes der Technik vermeiden, eine trockene und saubere Aufbewahrung der Teststreifen sicherstellen und ermöglichen, dass jederzeit korrekte Chargeninformationen der Teststreifen verwendet werden.

### Lösung

Diese Aufgabe wird durch die Erfindung mit den Merkmalen der unabhängigen Ansprüche gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt dieser Beschreibung gemacht.

Es wird ein tragbares analytisches Messsystem zur Messung von Stoffkonzentrationen in fluiden Medien vorgeschlagen. Insbesondere kann es sich dabei um die Messung von Blutglukosekonzentrationen und/oder Blutfettkonzentrationen handeln. Alternativ oder zusätzlich können jedoch auch andere Stoffkonzentrationen ermittelt werden oder entsprechende andere Analysen durchgeführt werden, beispielsweise pH-Messungen oder ähnliche Analysen. Unter fluiden Medien sollen dabei insbesondere flüssige Proben oder auch gasförmige Proben zu verstehen sein.

Das Messsystem weist ein Teststreifenbehältnis zur Aufnahme mindestens eines Teststreifens auf. Bei diesen Teststreifen kann es sich beispielsweise um einen der oben erwähnten Teststreifen für eine elektrochemische und/oder photometrische Analyse flüssiger Proben handeln. Insbesondere kann es sich dabei um Teststreifen der oben beschriebenen Art mit einem Kapillarsystem und einer oder mehreren Elektroden sowie zusätzlichen chemischen Hilfsstoffen (zum Beispiel Enzymen, Mediatoren) handeln. Unter "Teststreifen" lassen sich dabei sinngemäß auch andere Testvehikel verstehen, insbesondere kleine Teströhrchen oder auch kontinuierliche Rollen von Teststreifen.

Das Teststreifenbehältnis weist mindestens eine Verschlusseinrichtung zum Verschließen des Teststreifenbehältnisses bzw. zum Öffnen des Teststreifenbehältnisses zum Zwecke der Entnahme eines oder mehrerer Teststreifen auf. Diese Verschlusseinrichtung kann auf verschiedene Weise ausgestaltet sein. Insbesondere kann diese Verschlusseinrichtung derart ausgestaltet sein, dass sich mittels der Verschlusseinrichtung das Teststreifenbehältnis hermetisch verschließen lässt. Auf diese Weise werden in dem Teststreifenbehältnis enthaltene Teststreifen optimal gegen Einwirkungen durch Luftfeuchtigkeit oder Luftsauerstoff beziehungsweise andere Verschmutzungen geschützt. Die Verschlusseinrichtung kann dabei beispielsweise so ausgestaltet sein, dass sie einen einfachen Stopfen aufweist, welcher zum Verschließen in eine Öffnung des Teststreifenbehältnisses eingepresst wird. Dieser Stopfen kann insbesondere lose sein oder auch, beispielsweise über eine flexible Verbindung, mit dem Teststreifenbehältnis verbunden sein, so dass die Wahrscheinlichkeit eines Verlusts des Stopfens in geöffnetem Zustand des Teststreifenbehältnisses verringert wird. Alternativ oder zusätzlich kann die Verschlusseinrichtung beispielsweise auch einen Deckel mit einem Scharnier aufweisen, wobei idealerweise in geschlossenem Zustand der Deckel das Teststreifenbehältnis hermetisch verschließt.

Weiterhin weist das Teststreifenbehältnis eine Zählvorrichtung zum Zählen einer Anzahl und/oder einer zeitlichen Dauer von Öffnungsvorgängen der Verschlusseinrichtung sowie einen Datenträger zum Speichern von chargenspezifischen Daten der Teststreifen und/oder von einer Anzahl und/oder zeitlichen Dauer von Öffnungsvorgängen auf. Die Zählvorrichtung kann beispielsweise eine Uhr aufweisen und eine entsprechende Vorrichtung (beispielsweise eine Vorrichtung, wie sie in der EP 0 101 812 B1 beschrieben ist), welche ein Öffnen der Verschlusseinrichtung registriert und beispielsweise bei jedem Öffnen eine Zählervariable um Eins erhöht. Auch kann die Vorrichtung, sobald ein Öffnen der Verschlusseinrichtung registriert wird, einen zeitlichen Zähler, beispielsweise eine Uhr, starten, welche dann bei anschließendem Schließen der Verschlusseinrichtung wieder gestoppt wird. Auf diese Weise lassen sich die Zeiten, während derer das Teststreifenbehältnis geöffnet ist, ermitteln. Insbesondere lassen sich auf diese Weise einzelne Zeiten oder auch Zeiten kumulativ bestimmen. Die so ermittelten Zeiten beziehungsweise die so ermittelte Anzahl von Öffnungsvorgängen der Verschlusseinrichtung können insbesondere auf dem Datenträger gespeichert werden. Weiterhin können auf dem Datenträger chargenspezifische Daten der Teststreifen, wie oben beschrieben, abgespeichert werden.

Weiterhin weist das Teststreifenbehältnis eine Datenübertragungsvorrichtung zum drahtlosen Übertragen von chargenspezifischen Daten und eine Anzahl und/oder zeitlichen Dauer von Öffnungsvorgängen an ein Analysemodul auf. Insbesondere können mittels dieser Datenübertragungsvorrichtung die auf dem Datenträger gespeicherten Daten übertragen werden. Die Datenübertragung an das Analysemodul soll dabei drahtlos erfolgen. Insbesondere kann die Datenübertragungsvorrichtung mindestens einen Transponder aufweisen. Unter einem Transponder kann dabei insbesondere ein radio frequency identification (RFID) tag oder eine einen solchen RFID tag aufweisende Vorrichtung verstanden werden. Insbesondere kann der Transponder auch einen Mikroprozessor und auch einen eigenen Speicher aufweisen. Weiterhin weist der Transponder mindestens eine Antenne zum Übertragen von Daten auf, beispielsweise eine auf ein Substrat aufgedruckte Kupferspule. Weiterhin kann der Transponder eine eigene Energiequelle aufweisen, beispielsweise eine Batterie. Alternativ oder zusätzlich kann der Transponder auch extern mit Energie versorgt werden, beispielsweise durch Empfang entsprechender elektromagnetischer Wellen, welche beispielsweise von einem Analysemodul ausgesandt werden. Der Transponder kann ausgestaltet sein, um Daten lediglich zu senden, kann jedoch auch ausgestaltet sein, um Daten zu senden und Daten zu empfangen. Insbesondere kann der Transponder beispielsweise in einem Frequenzbereich von ca. 900 MHz senden.

Weiterhin weist das Messsystem ein Analysemodul mit einer Vorrichtung zu Messen von Stoffkonzentrationen in fluiden Medien mittels eines Teststreifens auf. Hierbei kann es sich beispielsweise um ein Analysemodul handeln, welches im Wesentlichen aus dem Stand der Technik bekannte Funktionen eines Blutglukosekonzentrationsmessgerätes aufweist. Insbesondere kann es sich dabei um ein Analysemodul handeln, welches, wie oben beschrieben, Blutglukosekonzentrationen mittels eines elektrochemischen oder optischen Messverfahrens bestimmt. Derartige Analysemodule sind aus dem Stand der Technik bekannt, so dass die weitere Funktionalität dieser Module hier nicht weiter beschrieben werden soll.

Das erfindungsgemäße Analysemodul weist weiterhin eine Datenempfangsvorrichtung zum drahtlosen Empfang von chargenspezifischen Daten und einer Anzahl und/oder zeitlichen Dauer von Öffnungsvorgängen auf. Mit dieser Datenempfangsvorrichtung können beispielsweise die von der Datenübertragungsvorrichtung im Teststreifenbehältnis ausgesandten Informationen (siehe oben) empfangen werden. Dabei kann die Datenempfangsvorrichtung als reine Empfangsvorrichtung ausgestaltet sein, es kann sich jedoch auch um eine Vorrichtung handeln, welche sowohl eine Empfangsvorrichtung als auch eine Sendevorrichtung aufweist. So kann die Datenempfangsvorrichtung beispielsweise eine Sendevorrichtung aufweisen, welche zunächst über ein drahtloses Signal (welches beispielsweise in regelmäßigen Abständen ausgesandt wird) eine Datenübertragungsvorrichtung in einem Teststreifenbehältnis, welches sich möglicherweise in der Nähe des Analysemoduls befindet, zum drahtlosen Übertragen von Daten anregt. Anschließend werden diese Daten vom Analysemodul mittels der Datenempfangsvorrichtung empfangen. Weiterhin kann, wie oben beschrieben, die Datenempfangsvorrichtung vorzugsweise gleichzeitig auch zur Energieversorgung der Datenübertragungsvorrichtung in einem Teststreifenbehältnis über elektromagnetische Wellen genutzt werden. Unter elektromagnetischen Wellen für den Austausch von Daten und Energie zwischen dem Analysemodul und dem Teststreifenbehältnis können dabei beispielsweise Hochfrequenzwellen oder auch elektromagnetische Wellen im infraroten Spektralbereich verstanden werden. Auch andere Spektralbereiche des elektromagnetischen Spektrums sind denkbar.

Das Messsystem kann insbesondere so ausgestaltet sein, dass das Analysemodul eine Warmvorrichtung zum Warnen eines Benutzers aufweist. Wird beispielsweise anhand der Übertragung von Daten vom Teststreifenbehältnis festgestellt, dass das Teststreifenbehältnis zu oft, d.h. öfters als eine vorgegebene Anzahl, geöffnet worden ist, so kann eine Warnung an einen Benutzer des Messsystems ausgegeben werden. Diese Warnung kann beispielsweise in Form eines akustischen Signals oder in Form einer optischen Anzeige, beispielsweise eine Anzeige durch eine Leuchtdiode oder durch eine Anzeige auf einem Display, erfolgen. Weiterhin kann, ebenfalls beispielsweise anhand der vom Teststreifenbehältnis übertragenen Daten, ermittelt werden, ob das Teststreifenbehältnis zu lange geöffnet war. Dabei kann es sich um eine maximale zeitliche Gesamtdauer von Öffnungsvorgängen des Teststreifenbehältnisses oder auch um die Dauer eines einzelnen Öffnungsvorgangs handeln. Auf diese Weise wird verhindert, dass der Benutzer für die Messung von Stoffkonzentrationen Teststreifen einsetzt, welche zu lange der Atmosphäre beziehungsweise der Luftfeuchtigkeit ausgesetzt waren. Alternativ oder zusätzlich können auch andere Warmfunktionen wahrgenommen werden. Beispielsweise kann aus der maximalen Anzahl von Öffnungsvorgängen geschlossen werden, beispielsweise unter der Annahme, dass bei jedem Öffnungsvorgang ein Teststreifen aus dem Teststreifenbehältnis entnommen wird, dass sich nur noch eine bestimmte Anzahl von Teststreifen in dem Teststreifenbehältnis befindet. Beispielsweise kann diese Anzahl verbleibender Teststreifen über eine Anzeigevorrichtung, beispielsweise ein Display im Analysemodul, dem Nutzer des Messsystems mitgeteilt werden. Ist das Teststreifenbehältnis leer, so kann ebenfalls eine Warnmeldung ausgegeben werden. Auch andere Warnsignale können erzeugt werden, beispielsweise wenn das Teststreifenbehältnis einen Feuchtigkeitssensor enthält, der eine Gesamtbeaufschlagung der Teststreifen mit Luftfeuchtigkeit in dem Teststreifenbehältnis misst, diese Information über die Datenübertragungsvorrichtung an das Analysemodul übertragen wird, und diese dann dahingehend ausgewertet wird, ob die Teststreifen insgesamt zu lange einer bestimmten maximalen Luftfeuchtigkeit ausgesetzt waren.

Die Messung von Stoffkonzentrationen in fluiden Medien mittels der Teststreifen kann dann im Analysemodul, insbesondere unter Zuhilfenahme der vom Teststreifenbehältnis übertragenen Daten, erfolgen. So kann insbesondere die Chargeninformation beziehungsweise die chargenspezifischen Daten zum Berechnen der Stoffkonzentrationen genutzt werden. Zu diesem Zweck kann das Analysemodul eine entsprechende Auswertevorrichtung aufweisen. Auch andere Daten, wie beispielsweise Daten über die aufgenommene Feuchtigkeit in den Teststreifen, können in die Berechnung der Stoffkonzentrationen einfließen. So kann beispielsweise eine Kalibration durchgeführt werden, in welcher berücksichtigt wird, wie die Berechnung der Stoffkonzentration zu modifizieren ist, wenn ein Teststreifen eine bestimmte Menge an Luftfeuchtigkeit aufgenommen hat. Diese Informationen können im Analysemodul, beispielsweise in der Auswertevorrichtung, zum Beispiel in einer elektronischen Tabelle (z. B. einer lookup table) hinterlegt sein.

Weiterhin wird eine Verwendung eines erfindungsgemäßen Teststreifenbehältnisses (wie oben beschrieben) in einem Messsystem zur Messung von Stoffkonzentrationen in fluiden Medien vorgeschlagen. Schließlich wird ein Verfahren zur Messung von Stoffkonzentrationen in fluiden Medien mittels eines erfindungsgemäßen Messsystems (wie oben beschrieben) vorgeschlagen.

Das beschriebene Messsystem und das entsprechende Verfahren zur Nutzung des Messsystems weisen gegenüber herkömmlichen Systemen und Verfahren zahlreiche Vorteile auf. Ein wesentlicher Vorteil liegt insbesondere darin, dass chargenspezifische Daten der Teststreifen, insbesondere Kalibrationsdaten und eventuell weitere Informationen, automatisch vom Teststreifenbehältnis an das Analysemodul übertragen werden. Ein Eingriff des Benutzers, beispielsweise in Form eines Auslesens eines ROM-Keys oder eines Strichcodes, ist nicht erforderlich. Dadurch wird die Sicherheit der Handhabung des Messsystems erheblich erhöht, da Fehler bei der Eingabe der chargenspezifischen Informationen in das Analysemodul stark verringert beziehungsweise unwahrscheinlicher werden. Auch besteht nicht mehr die Gefahr, dass beispielsweise lose am Teststreifenbehältnis angebrachte Datenträger, insbesondere ROM-Keys, beim Handhaben des Teststreifenbehältnisses verloren gehen. Der Datenträger im Teststreifenbehältnis kann nicht verloren gehen, da er Bestandteil des Teststreifenbehältnisses ist, beispielsweise in die Verschlusseinrichtung integriert ist, welche wiederum zum Verschließen des Teststreifenbehältnisses benötigt wird.

Weiterhin wird die Sicherheit der Messung von Stoffkonzentrationen durch Kenntnis des Zustandes der Teststreifen stark verbessert. Die Information darüber, wie oft und wie lange das Teststreifenbehältnis geöffnet war und wie oft dementsprechend die Teststreifen der Luft und der Luftfeuchtigkeit ausgesetzt waren, verhindert beziehungsweise verringert Abweichungen der Messungen und Fehlmessungen, welche, wie oben beschrieben, zu fatalen Folgen für den Nutzer beziehungsweise Patienten führen könnten.

Im Folgenden wird die Erfindung anhand von Ausführungsbeispielen näher beschrieben. Die Erfindung ist jedoch nicht auf die dargestellten Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigt:
- Figur 1: ein Ausführungsbeispiel eines tragbaren analytischen Messsystems zur Messung von Stoffkonzentrationen in fluiden Medien mit einem Teststrei- fenbehältnis und einem Analysemodul; und
- Figur 2: einen Ablaufplan eines Verfahrens zur Messung von Stoffkonzentrationen in fluiden Medien mittels eines erfindungsgemäßen Messsystems.

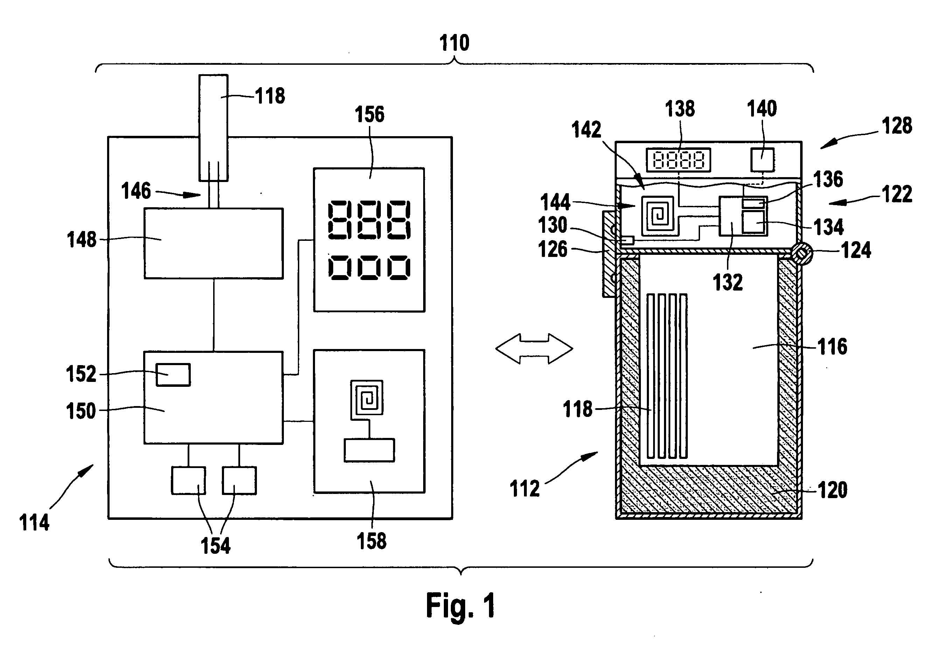

In Figur 1 ist ein tragbares analytisches Messsystem 110 zur Messung von Stoffkonzentrationen in fluiden Medien, insbesondere zur Messung von Blutglukosekonzentrationen, dargestellt. Das Messsystem 110 weist ein Teststreifenbehältnis 112 und ein Analysemodul 114 auf. Das Teststreifenbehältnis 112 weist einen Vorratsraum 116 auf, in welchem eine Anzahl von Teststreifen 118 gelagert ist. Bei den Teststreifen 118 handelt es sich in diesem Ausführungsbeispiel um dem Stand der Technik entsprechende Teststreifen 118 zur Blutglukosekonzentrationsmessung mittels eines elektrochemischen Messverfahrens (siehe oben).

Die Teststreifen 118 sind feuchtigkeitsempfindlich. Aus diesem Grund ist in den Vorratsraum 116 ein Trockenmittel 120 eingebracht, welches die Luftfeuchtigkeit im Inneren des Vorratsraums 116 reduziert. Weiterhin weist der Vorratsraum 116 eine Verschlusseinrichtung 122 auf. Die Verschlusseinrichtung 122 weist ein Scharnier 124, einen Verschluss 126 und einen Deckel 128 auf. Mittels der Verschlusseinrichtung 122 kann der Vorratsraum 116 luftdicht verschlossen werden, indem der Deckel 128 mittels des Scharniers 124 geschlossen und mittels des Verschlusses 126 fixiert wird. Der Rand des Deckels 128 kann zusätzlich mit einem O-Ring versehen sein, um den Vorratsraum 116 zusätzlich abzudichten.

Der Deckel 128 ist in diesem Ausführungsbeispiel als sogenannter elektronischer Deckel (eCap) 128 ausgestaltet, ähnlich der in der EP 0 101 812 B1 beschriebenen Vorrichtung. Zu diesem Zweck weist der Deckel 128 einen Öffnungssensor 130 auf, welcher registriert, ob die Verschlusseinrichtung 122 geöffnet oder geschlossen ist. Dieser Öffnungssensor 130 kann verschiedenartig ausgestaltet sein und kann beispielsweise einen oder mehrere elektrische Kontakte sowie ein nachgiebiges Glied umfassen, wobei bei Schließen der Verschlusseinrichtung 122 mittels des nachgiebigen Gliedes eine elektrische Verbindung zwischen den Kontakten hergestellt wird. Ein derartiger Öffnungssensor ist beispielsweise in der EP 0 101 812 B1 beschrieben und soll daher hier nicht mehr im Detail dargestellt werden.

Der Öffnungssensor 130 ist verbunden mit einem Mikroprozessor 132, welcher über eine Zählvorrichtung 134 (beispielsweise in Form eines elektronischen Taktgebers, insbesondere einer Uhr) verfügt. Weiterhin verfügt der Mikroprozessor 132 über einen Datenträger 136, beispielsweise in Form eines flüchtigen Speichers (RAM) oder auch eines Festspeichers, insbesondere eines EPROM's. Der Mikroprozessor 132 kann auch über mehrere Datenträger 136 verfügen, beispielsweise über einen flüchtigen Speicher zum fortlaufenden Abspeichern und Auslesen von Informationen über Öffnungsvorgänge der Verschlusseinrichtung 122, sowie einen festen Speicher in Form eines EPROM's zum Abspeichern und Auslesen von chargenspezifischen Informationen über die Teststreifen 118.

Weiterhin ist der Mikroprozessor 132 verbunden mit einem Anzeigeelement 138 in Form eines einfachen LC-Displays sowie mit einem Bedienelement 140, beispielsweise in Form eines oder mehrerer Druckknöpfe. Schließlich ist der Mikroprozessor 132 verbunden mit einer Sende- und Empfangsvorrichtung 142, welche in Figur 1 symbolisch als Spulenantenne dargestellt ist. Auch andere Antennenformen sind möglich. Neben der Spulenantenne kann die Sende- und Empfangsvorrichtung 142 jedoch noch eine Reihe weiterer Elemente umfassen (nicht dargestellt), beispielsweise entsprechende andere Komponenten eines elektrischen Schwingkreises wie Kondensatoren und Widerstände. Diese Sende- und Empfangsvorrichtung 142 kann insbesondere auch als gedruckte Schaltung ausgeführt sein. Mikroprozessor 132 und Sende- und Empfangsvorrichtung 142 bilden gemeinsam in diesem Ausführungsbeispiel einen Transponder 144.

Weiterhin weist das tragbare analytische Messsystem 110, wie oben beschrieben, ein Analysemodul 114 auf. Das Analysemodul 114 ist insbesondere ausgestaltet, um einen Teststreifen 118 aufzunehmen, wobei dieser Teststreifen 118 mittels eines Messelektrodensystems 146 elektrisch kontaktiert wird. Über eine geeignete Auswertevorrichtung 148, beispielsweise eine elektronische Schaltung zur Impedanz- oder Strom-Spannungs-Messung, können - wie oben beschrieben - Ladungskonzentrationen bestimmt werden und somit auf die Blutglukosekonzentration eines auf den Teststreifen 118 aufgebrachten Bluttropfens zurückgeschlossen werden. Derartige Vorrichtungen sind aus dem Stand der Technik bekannt und sind bereits heute in Form entsprechender Messgeräte kommerziell verfügbar.

Weiterhin ist die Auswertevorrichtung 148 verbunden mit einem Mikroprozessor 150. Der Mikroprozessor 150 verfügt über einen oder mehrere Datenspeicher 152, beispielsweise wiederum in Form von flüchtigen Speichern oder auch in Form von nicht-flüchtigen Speichern. Weiterhin ist der Mikroprozessor 150 verbunden mit Bedienelementen 154, beispielsweise in Form von Druckknöpfen, welche auf einer Benutzeroberfläche des Analysemoduls 114 angeordnet sind und über die ein Benutzer das Analysemodul 114 bedienen kann. Auch ein Anzeigeelement 156 ist im Analysemodul 114 vorgesehen, welches mit dem Mikroprozessor 150 verbunden ist. Über dieses Anzeigeelement 156, welches beispielsweise wiederum ein LC-Display aufweisen kann, kann ein Benutzer beispielsweise Ergebnisse der Blutglukosekonzentrationsmessung ablesen.

Weiterhin weist das Analysemodul 114 wiederum eine Sende- und Empfangsvorrichtung 158 auf, welche mit dem Mikroprozessor 150 verbunden ist. Die Sende- und Empfangsvorrichtung 158 ist in diesem Ausführungsbeispiel wiederum symbolisch durch eine Sendespule dargestellt, kann jedoch, wie auch im Fall des Teststreifenbehältnisses 112, wiederum über eine Reihe von zusätzlichen elektronischen Bauelementen verfügen (nicht dargestellt), insbesondere eine Reihe von Kondensatoren, Widerständen oder auch aktiven elektronischen Bauelementen sowie eigenen Mikroprozessoren.

Das beschriebene Messsystem 110 verfügt über eine Reihe von Funktionalitäten, welche auf die speziellen Bedürfnisse einer Überwachung einer Blutglukosekonzentration durch einen Patienten angepasst sind. So weist insbesondere das Teststreifenbehältnis 112 neben der Funktion einer trockenen und sauberen Lagerung der Teststreifen 118 im Vorratsraum 116 eine Reihe von Warn- und Informationsfunktionen auf. So registriert der Mikroprozessor 132 jeden mittels des Öffnungssensors 130 festgestellten Öffnungs- und Schließvorgang der Verschlusseinrichtung 122. Insbesondere kann der Mikroprozessor 132 ein akustisches (über einen nicht dargestellten Lautsprecher) oder auch ein optisches (zum Beispiel über eine Leuchtdiode oder das Anzeigeelement 138) Warnsignal erzeugen, wenn über den Öffnungssensor 130 festgestellt wird, dass die Verschlusseinrichtung 122 über einen längeren Zeitraum hinweg nicht geschlossen ist. Somit kann ein Benutzer des Teststreifenbehältnisses 112 sofort einschreiten und das Teststreifenbehältnis 112 entsprechend verschließen.

Weiterhin kann die Anzahl der Öffnungs- und Schließvorgänge der Verschlusseinrichtung 122 über die Zählvorrichtung 134 registriert werden. Diese Information kann zu verschiedenen Zwecken genutzt werden. Zum einen kann, unter der Annahme, dass bei jedem Öffnungsvorgang ein Teststreifen 118 aus dem Vorratsraum 116 entnommen wird, der "Füllstand" des Teststreifenbehältnisses 112 überwacht und an den Benutzer übermittelt werden. Beispielsweise kann die Anzahl der Teststreifen 118 von einer ursprünglichen Anzahl rückwärts gezählt werden, so dass ein Benutzer des Teststreifenbehältnisses 112 rechtzeitig gewarnt wird, wenn die Teststreifen 118 im Teststreifenbehältnis 112 zur Neige gehen. Insbesondere kann der Benutzer beispielsweise vorgeben, dass er bei einer bestimmten Mindestanzahl von Teststreifen 118 im Teststreifenbehältnis 112 gewarnt werden möchte, um rechtzeitig das Teststreifenbehältnis 112 wieder auffüllen zu können.

Weiterhin kann aus der Anzahl der Öffnungs- beziehungsweise Schließvorgänge der Verschlusseinrichtung 122 beziehungsweise aus der Gesamtdauer der Öffnungen auf eine Luftfeuchtigkeit im Vorratsraum 116 geschlossen werden. Beispielsweise können die mittels der Zählvorrichtung 134 ermittelten Zeitspannen, während derer das Teststreifenbehältnis 112 geöffnet war, vom Mikroprozessor 132 zu einer Gesamtdauer einer Öffnung addiert werden. Wird eine bestimmte maximale Gesamtdauer überschritten, so kann beispielsweise ein Warnsignal an einen Benutzer ausgegeben werden. Zusätzlich oder alternativ kann der Mikroprozessor 132 auch mit einem Feuchtigkeitssensor (nicht dargestellt) im Vorratsraum 116 des Teststreifenbehältnisses 112 verbunden sein, welcher permanent die Luftfeuchtigkeit im Vorratsraum 116 überwacht und an den Mikroprozessor übermittelt.

Der Mikroprozessor 132 kann über das Bedienelement 140 vom Benutzer programmiert werden, wobei in Gegenrichtung über das Anzeigeelement 138 Informationen an den Benutzer vermittelt werden. So kann der Benutzer beispielsweise auch vorgeben, dass er vom Mikroprozessor 132 in regelmäßigen Zeitabständen, welche vom Benutzer definierbar sind, daran erinnert werden möchte (wiederum beispielsweise über ein optisches oder akustisches Signal), dass eine Blutglukosekonzentrationsmessung durchgeführt werden muss.

Anstelle einer direkten Kommunikation des Mikroprozessors 132 mit dem Benutzer über optische oder akustische Signale können sämtliche Funktionalitäten beispielsweise auch vom Analysemodul 114 übernommen werden. Zu diesem Zweck können sämtliche dem Mikroprozessor 132 des Teststreifenbehältnisses 112 zur Verfügung stehenden Informationen über die Sende- und Empfangsvorrichtung 142 an das Analysemodul 114 übertragen werden. Insbesondere kann dies dadurch erfolgen, dass die Sende- und Empfangsvorrichtung 158 im Analysemodul 114 in regelmäßigen Abständen Signale aussendet, welche von einem Transponder 144 eines in der Nähe befindlichen Teststreifenbehältnisses 112 empfangen werden können. Seinerseits kann dann wiederum der Transponder 144 des Teststreifenbehältnisses 112 ein entsprechendes Signal an das Analysemodul 114 zurücksenden. Auf diese Weise erkennt das Messsystem 110, dass sich ein Teststreifenbehältnis 112 und ein Analysemodul 114 in ausreichender räumlicher Nähe zueinander befinden und Daten austauschen können. Anschließend kann ein entsprechender Datenaustausch zwischen dem Mikroprozessor 132 des Teststreifenbehältnisses 112 und dem Mikroprozessor 150 des Analysemoduls 114 stattfinden. So kann beispielsweise das Analysemodul 114 vom Teststreifenbehältnis 112 entsprechende Informationen anfordern, beispielsweise Informationen darüber, ob sich noch Teststreifen 118 im Vorratsraum 116 des Teststreifenbehältnisses 112 befinden und gegebenenfalls in welchem Zustand (bezüglich Aufnahme von Luftfeuchtigkeit oder ähnlichem) sich diese Teststreifen 118 befinden. Entsprechende Informationen können dann vom Mikroprozessor 150 des Analysemoduls 114 dem Benutzer, beispielsweise über das Anzeigeelement 156 des Analysemoduls 114 oder über geeignete akustische Signale, vermittelt werden. So kann beispielsweise auch das Analysemodul 114 einen Benutzer darauf hinweisen, dass neue Teststreifen 118 in den Vorratsraum 116 des Teststreifenbehältnisses 112 eingebracht werden müssen, oder dass die Zeit für eine erneute Bestimmung der Blutglukosekonzentration gekommen ist. Da üblicherweise das Analysemodul 114 über einen leistungsfähigeren Mikroprozessor 150 mit umfangreicheren Bedienelementen 154 verfügt, kann beispielsweise auch der Mikroprozessor 132 im Teststreifenbehältnis 112 am Analysemodul 114 programmiert werden. Dies hat den Vorteil einer erhöhten Benutzerfreundlichkeit und einer Erweiterung der Funktionalitäten des Messsystems 110.

Weiterhin kann auch automatisch eine Übertragung von Chargeninformationen zwischen dem Teststreifenbehältnis 112 und dem Analysemodul 114 erfolgen. Zu diesem Zweck können im Datenträger 136 des Mikroprozessors 132, beispielsweise in einem EPROM, entsprechende chargenspezifische Informationen über die Teststreifen 118 im Teststreifenbehältnis 112 gespeichert werden. Dabei kann auf verschiedene Weise vorgegangen werden. Beispielsweise kann das Teststreifenbehältnis 112 als Einwegbehältnis ausgestaltet sein, wobei ein Benutzer beispielsweise in einer Apotheke das vollständige Teststreifenbehältnis 112 erwirbt. In diesem Fall sind chargenspezifische Informationen über die Teststreifen 118 bereits beim Erwerb des Teststreifenbehältnisses 112 im Datenträger 136 des elektronischen Deckels 128 gespeichert. Auch ein entsprechendes Pfandsystem wäre denkbar. Dabei kann das beispielsweise ein zurückgegebenes, leeres Teststreifenbehältnis 112, beispielsweise in einer Apotheke oder bei einem Pharma-Großhändler, wieder mit Teststreifen 118 befüllt werden, wobei dann entweder der Transponder 144 oder Bestandteile desselben komplett ausgetauscht wird, oder alternativ auch neue chargenspezifische Informationen über die Teststreifen 118 über die Sende- und Empfangsvorrichtung 142 in den Mikroprozessor 132 eingespielt werden. Alternativ kann ein Benutzer beim Erwerb von Teststreifen 118 auch einen entsprechenden Transponder 144 oder Bestandteile desselben erhalten, zusammen mit den Anweisungen, diesen Transponder 144 in den Deckel 122 des Teststreifenbehältnisses 112 einzufügen.

Die Übertragung von chargenspezifischen Informationen aus dem Datenträger 136 des Mikroprozessors 132 über die Sende- und Empfangsvorrichtung 142 an das Analysemodul 114 kann mit dem dargestellten Messsystem 110 automatisch erfolgen. Beispielsweise kann das Analysemodul 114, sobald (siehe oben) festgestellt wird, dass sich ein entsprechendes Teststreifenbehältnis 112 in einer für eine Datenübertragung geeigneten Nähe befindet, vom Teststreifenbehältnis 112 mittels eines speziellen Anforderungssignals die entsprechenden chargenspezifischen Informationen über die Teststreifen 118 anfordern.

Nicht dargestellt in Figur 1 sind die jeweiligen Energieversorgungen des Analysemoduls 114 und des Teststreifenbehältnisses 112. Das Analysemodul 114 kann beispielsweise über einen Satz von Batterien mit elektrischer Energie versorgt werden. Komplexer ist die Energieversorgung des Teststreifenbehältnisses 112 mit elektrischer Energie, da insbesondere im Deckel 128 nur geringer Platz für eine entsprechende Anordnung von Batterien zur Verfügung steht. Dennoch kann beispielsweise in den Deckel 128 eine entsprechende elektrische Knopfzelle zur Energieversorgung eingebracht werden. Alternativ oder zusätzlich kann jedoch auch ein Energieversorgungssystem angewandt werden, bei welchem die Energieversorgung des Deckels 128 beispielsweise über elektromagnetische Wellen erfolgt, welche vom Analysemodul 114, speziell von der Sende- und Empfangsvorrichtung 158, an das Teststreifenbehältnis 112 übertragen werden. Diese elektromagnetischen Wellen können insbesondere beispielsweise von der Sende- und Empfangsvorrichtung 142 des Deckels 128 empfangen werden und beispielsweise zum Aufladen einer entsprechenden Energiequelle, beispielsweise eines Kondensators, verwendet werden. Ein derartiges Prinzip wird bereits heute in verschiedenen Transponder-Vorrichtungen auf anderen technischen Gebieten eingesetzt. Auch eine Trennung der Funktionalitäten im Deckel 128 ist denkbar, beispielsweise eine getrennte Energieversorgung von Sende- und Empfangsvorrichtung 142 und Mikroprozessor 132. So kann beispielsweise das einfache Anfordern von Daten durch das Analysemodul 114 durch ein entsprechendes Signal angeregt werden, welches von der Sende- und Empfangsvorrichtung 158 des Analysemoduls 114 ausgesandt wird, wobei diese elektromagnetischen Wellen von der Sende- und Empfangsvorrichtung 142 des Teststreifenbehältnisses 112 empfangen werden, wobei dort eine entsprechende Energiequelle aufgeladen wird, um dann entsprechend die angeforderten Daten an das Analysemodul 114 zurücksenden zu können.

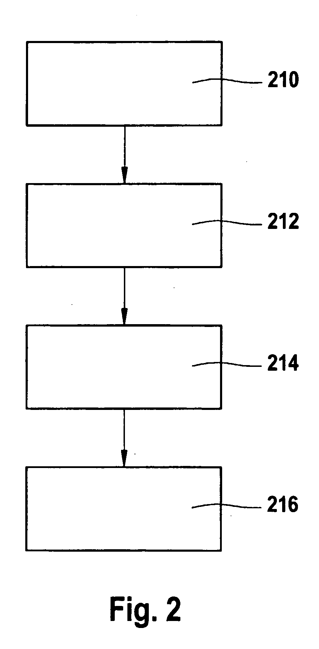

In Figur 2 ist ein Verfahren zur Messung von Stoffkonzentrationen in fluiden Medien mittels eines Messsystems 110, beispielsweise des in Figur 1 beschriebenen Messsystems 110, dargestellt. Die dargestellten Schritte müssen nicht notwendigerweise in der abgebildeten Reihenfolge durchgeführt werden, und es können auch zusätzliche, in Figur 2 nicht dargestellte Schritte durchgeführt werden.

Zunächst wird in Verfahrensschritt 210 eine Anzahl und/oder zeitliche Dauer von Öffnungsvorgängen einer Verschlusseinrichtung 122 mittels einer Zählvorrichtung 134 erfasst und auf einem Datenträger 136 gespeichert. Anschließend werden in Verfahrensschritt 212 chargenspezifische Daten und eine Anzahl und/oder zeitliche Dauer von Öffnungsvorgängen an ein Analysemodul 114 übertragen. Stellt das Analysemodul 114 durch Vergleich dieser Daten fest, dass eine maximale Anzahl von Öffnungsvorgängen, welche beispielsweise in einem Datenspeicher 152 eines Mikroprozessors 150 im Analysemodul 114 gespeichert sein kann und durch einen Benutzer, beispielsweise über die Bedienelemente 154 eingestellt werden kann, oder wenn eine maximale zeitliche Gesamtdauer von Öffnungsvorgängen des Teststreifenbehältnisses 112 überschritten worden ist, so erfolgt im optionalen Verfahrensschritt 214 eine entsprechende Warnung an einen Benutzer des Analysemoduls 114, beispielsweise in Form einer optischen Anzeige auf dem Display 156 oder in Form einer akustischen Anzeige. Anschließend wird in Verfahrensschritt 216 mittels eines Teststreifens 118 am Analysemodul 114 eine Stoffkonzentration in einem fluiden Medium, insbesondere eine Blutglukosekonzentration, bestimmt.

### Bezugszeichenliste

- 110: tragbares analytisches Messsystem
- 112: Teststreifenbehältnis
- 114: Analysemodul
- 116: Vorratsraum
- 118: Teststreifen
- 120: Trockenmittel
- 122: Verschlusseinrichtung
- 124: Scharnier
- 126: Verschluss
- 128: Deckel
- 130: Öffnungssensor
- 132: Mikroprozessor
- 134: Zählvorrichtung
- 136: Datenträger
- 138: Anzeigeelement
- 140: Sende- und Empfangsvorrichtung
- 144: Transponder
- 146: Messelektrodensystem
- 148: Auswertevorrichtung
- 150: Mikroprozessor
- 152: Datenspeicher
- 154: Bedienelement
- 156: Anzeigeelement
- 158: Sende- und Empfangsvorrichtung

- 210: Erfassen und Abspeichern einer Anzahl von Öffnungsvorgängen
- 212: Übertragung von Daten an Analysemodul 14
- 214: Warnung an Benutzer
- 216: Blutglukosekonzentrationsmessung

## Patentansprüche

1. Tragbares analytisches Messsystem (110) zur Messung von Stoffkonzentrationen in fluiden Medien mit
a) einem Teststreifenbehältnis (112) zur Aufnahme mindestens eines Teststreifens (118), wobei das Teststreifenbehältnis (112) folgendes aufweist:
- eine Verschlusseinrichtung (122) zum Verschließen des Teststreifenbehältnisses (112) bzw. zum Öffnen des Teststreifenbehältnisses (112) zum Zwecke der Entnahme eines oder mehrerer Teststreifen (118),
- eine Zählvorrichtung (134) zum Zählen einer Anzahl und/oder zeitlichen Dauer von Öffnungsvorgängen der Verschlusseinrichtung (122),
- einen Datenträger (136) zum Speichern von chargenspezifischen Daten der Teststreifen (118) und/oder von einer Anzahl und/oder zeitlichen Dauer von Öffnungsvorgängen,
- eine Datenübertragungsvorrichtung (144) zum drahtlosen Übertragen von chargenspezifischen Daten und einer Anzahl und/oder zeitlichen Dauer von Öffnungsvorgängen an ein Analysemodul (114); und
b) einem Analysemodul (114), wobei das Analysemodul (114) folgendes aufweist:
- eine Vorrichtung (148, 150) zum Messen von Stoffkonzentrationen in fluiden Medien mittels eines Teststreifens (118),
- eine Datenempfangsvorrichtung (158) zum drahtlosen Empfang von chargenspezifischen Daten und einer Anzahl und/oder zeitlichen Dauer von Öffnungsvorgängen.

2. Messsystem (110) gemäß dem vorhergehenden Anspruch, **dadurch gekennzeichnet, dass** die Datenübertragungsvorrichtung (144) mindestens einen Transponder (144) aufweist.

3. Messsystem (110) gemäß einem der beiden vorhergehenden Ansprüche, **dadurch gekennzeichnet, dass** das Analysemodul (114) eine Warmvorrichtung (150, 156) zum Warnen eines Benutzers bei Überschreiten einer maximalen Anzahl von Öffnungsvorgängen und/oder bei Überschreiten einer maximalen zeitlichen Gesamtdauer von Öffhungsvorgängen des Teststreifenbehältnisses (112) aufweist.

4. Messsystem (110) gemäß einem der vorhergehenden Ansprüche, **dadurch gekennzeichnet, dass** die Vorrichtung (148, 150) zum Messen von Stoffkonzentrationen in fluiden Medien eine Auswertevorrichtung (150) zum Berechnen der Stoffkonzentrationen in Abhängigkeit der chargenspezifischen Daten der Teststreifen (118) aufweist.

5. Messsystem (110) gemäß einem der vorhergehenden Ansprüche, **dadurch gekennzeichnet, dass** das Teststreifenbehältnis (112) in einem geschlossenen Zustand durch die Verschlusseinrichtung (122) hermetisch abgedichtet ist.

6. Messsystem (110) gemäß einem der vorhergehenden Ansprüche, **dadurch gekennzeichnet, dass** die Vorrichtung (148, 150) zum Messen von Stoffkonzentrationen in fluiden Medien eine Vorrichtung (148, 150) zum Messen von Blutglukosekonzentrationen und/oder Blutfettkonzentrationen aufweist.

7. Messsystem (110) gemäß dem vorhergehenden Anspruch, **dadurch gekennzeichnet, dass** die Vorrichtung (148, 150) zum Messen von Blutglukosekonzentrationen und/oder Blutfettkonzentrationen eine elektrochemische Messvorrichtung (148) und/oder eine optische Messvorrichtung aufweist.

8. Verwendung eines Teststreifenbehältnisses (112) zur Aufnahme mindestens eines Teststreifens (118) mit einer Verschlusseinrichtung (122) zum Öffnen des Teststreifenbehältnisses (112) zum Zwecke der Entnahme eines oder mehrerer Teststreifen (118), einer Zählvorrichtung (134) zum Zählen einer Anzahl und/oder zeitlichen Dauer von Öffnungsvorgängen der Verschlusseinrichtung (122), einem Datenträger (136) zum Speichern von chargenspezifischen Daten der Teststreifen (118) und/oder von einer Anzahl und/oder zeitlichen Dauer von Öffnungsvorgängen, einer Datenübertragungsvorrichtung (144) zum drahtlosen Übertragen von chargenspezifischen Daten und einer Anzahl und/oder zeitlichen Dauer von Öffnungsvorgängen an ein Analysemodul (114) in einem Messsystem (110) zur Messung von Stoffkonzentrationen in fluiden Medien.

9. Verfahren zur Messung von Stoffkonzentrationen in fluiden Medien mittels eines Messsystems (110) gemäß einem der vorhergehenden, auf ein Messsystem (110) gerichteten Ansprüche, **gekennzeichnet durch** folgende Schritte:
a) eine Anzahl und/oder zeitlichen Dauer von Öffnungsvorgängen wird mittels der Zählvorrichtung (134) erfasst und auf dem Datenträger (136) gespeichert;
b) chargenspezifische Daten und eine Anzahl und/oder zeitliche Dauer von Öffnungsvorgängen werden drahtlos an das Analysemodul (114) übertragen; und
c) mittels eines Teststreifens (118) wird eine Stoffkonzentration in einem fluiden Medium bestimmt.

10. Verfahren gemäß dem vorhergehenden Anspruch mit zusätzlich folgendem Schritt:
d) der Ausgabe einer Warnung an einen Benutzer durch das Analysemodul (114) bei Feststellung des Überschreitens einer maximalen Anzahl von Öffnungsvorgängen und/oder einer maximalen zeitlichen Gesamtdauer von Öffnungsvorgängen des Teststreifenbehältnisses (112).

## Claims

1. Portable analytical measurement system (110) for measuring substance concentrations in liquid media, with
a) a test strip container (112) for receiving at least one test strip (118), said test strip container (112) comprising the following:
- a sealing device (122) for closing the test strip container (112) or for opening the test strip container (112) for the purpose of removing one or more test strips (118),
- a counter device (134) for counting a number and/or time duration of opening processes of the sealing device (122),
- a data carrier (136) for storage of batch-specific data of the test strips (118) and/or of a number and/or time duration of opening processes,
- a data transmission device (144) for wireless transmission of batch-specific data and of a number and/or time duration of opening processes to an analysis module (114), and
b) an analysis module (114), said analysis module (114) comprising the following:
- a device (148, 150) for measuring substance concentrations in liquid media by means of a test strip (118),
- a data-receiving device (158) for wireless reception of batch-specific data and of a number and/or time duration of opening processes.

2. Measurement system (110) according to the preceding claim, **characterized in that** the data transmission device (144) comprises at least one transponder (144).

3. Measurement system (110) according to either of the two preceding claims, **characterized in that** the analysis module (114) comprises a warning device (150, 156) for warning a user when a maximum number of opening processes has been exceeded and/or when a maximum overall time duration of opening processes of the test strip container (112) has been exceeded.

4. Measurement system (110) according to one of the preceding claims, **characterized in that** the device (148, 150) for measuring substance concentrations in liquid media comprises an evaluation device (150) for calculating the substance concentration as a function of the batch-specific data of the test strips (118).

5. Measurement system (110) according to one of the preceding claims, **characterized in that** the test strip container (112), in a closed state, is sealed off hermetically by the sealing device (122).

6. Measurement system (110) according to one of the preceding claims, **characterized in that** the device (148, 150) for measuring substance concentrations in liquid media comprises a device (148, 150) for measuring blood glucose concentrations and/or blood fat concentrations.

7. Measurement system (110) according to the preceding claim, **characterized in that** the device (148, 150) for measuring blood glucose concentrations and/or blood fat concentrations comprises an electrochemical measurement device (148) and/or an optical measurement device.

8. Use of a test strip container (112) for receiving at least one test strip (118), with a sealing device (122) for opening the test strip container (112) for the purpose of removing one or more test strips (118), with a counter device (134) for counting a number and/or time duration of opening processes of the sealing device (122), with a data carrier (136) for storage of batch-specific data of the test strips (118) and/or of a number and/or time duration of opening processes, and with a data transmission device (144) for wireless transmission of batch-specific data and of a number and/or time duration of opening processes to an analysis module (114), in a measurement system (110) for measuring substance concentrations in liquid media.

9. Method for measuring substance concentrations in liquid media by means of a measurement system (110) according to one of the preceding claims directed at a measurement system (110), said method being **characterized by** the following steps:
a) a number and/or time duration of opening processes is recorded by means of the counter device (134) and stored on the data carrier (136);
b) batch-specific data and a number and/or time duration of opening processes are sent by wireless transmission to the analysis module (114), and
c) a substance concentration in a liquid medium is determined by means of a test strip (118).

10. Method according to the preceding claim and additionally comprising the following step:
d) the analysis module (114) outputs a warning to a user when it determines that a maximum number of opening processes has been exceeded and/or a maximum overall time duration of opening processes of the test strip container (112) has been exceeded.

## Revendications

1. Système de mesure analytique portable (110) pour mesurer des concentrations de substances dans des milieux liquides, avec
a) un réservoir de bandelettes de test (112) destiné à recevoir au moins une bandelette de test (118), dans lequel le réservoir de bandelettes de test (112) comprend les éléments suivants:
- un dispositif de fermeture (122) pour la fermeture du réservoir de bandelettes de test (112) ou pour l'ouverture du réservoir de bandelettes de test (112) afin de prélever une ou plusieurs bandelettes de test (118),
- un dispositif de comptage (134) pour le comptage d'un nombre et/ou d'une durée temporelle d'opérations d'ouverture du dispositif de fermeture (122),
- un support de données (136) pour le stockage de données spécifiques de charges des bandelettes de test (118) et/ou d'un nombre et/ou d'une durée temporelle d'opérations d'ouverture,
- un dispositif de transfert de données (144) pour le transfert sans fil de données spécifiques de charges et d'un nombre et/ou d'une durée temporelle d'opérations d'ouverture à un module d'analyse (114); et
b) un module d'analyse (114), dans lequel le module d'analyse (114) comprend les éléments suivants:
- un dispositif (148, 150) pour la mesure des concentrations de substances dans des milieux liquides au moyen d'une bandelette de test (118),
- un dispositif de réception de données (158) pour la réception sans fil de données spécifiques de charges et d'un nombre et/ou d'une durée temporelle d'opérations d'ouverture.

2. Système de mesure (110) selon la revendication précédente, **caractérisé en ce que** le dispositif de transfert de données (144) comprend au moins un transpondeur (144).

3. Système de mesure (110) selon l'une quelconque des deux revendications précédentes, **caractérisé en ce que** le module d'analyse (114) comprend un dispositif d'avertissement (150, 156) pour avertir un opérateur en cas de dépassement d'un nombre maximal d'opérations d'ouvertures et/ou en cas de dépassement d'une durée temporelle totale maximale d'opérations d'ouverture du réservoir de bandelettes de test (112).

4. Système de mesure (110) selon l'une quelconque des revendications précédentes, **caractérisé en ce que** le dispositif (148, 150) pour la mesure de concentrations de substances dans des milieux liquides comprend un dispositif d'évaluation (150) pour le calcul des concentrations de substances en fonction des données spécifiques de charges des bandelettes de test (118).

5. Système de mesure (110) selon l'une quelconque des revendications précédentes, **caractérisé en ce que** le réservoir de bandelettes de test (112) est, à l'état fermé, clos de façon hermétiquement étanche au moyen du dispositif de fermeture (122).

6. Système de mesure (110) selon l'une quelconque des revendications précédentes, **caractérisé en ce que** le dispositif (148, 150) pour la mesure de concentrations de substances dans des milieux liquides comprend un dispositif (148, 150) pour la mesure de concentrations de glucose dans le sang et/ou de concentrations de lipides dans le sang.

7. Système de mesure (110) selon la revendication précédente, **caractérisé en ce que** le dispositif (148, 150) pour la mesure de concentrations de glucose dans le sang et/ou de concentrations de lipides dans le sang comprend un dispositif de mesure électrochimique (148) et/ou un dispositif de mesure optique.

8. Utilisation d'un réservoir de bandelettes de test (112) destiné à recevoir au moins une bandelette de test (118), avec un dispositif de fermeture (122) pour l'ouverture du réservoir de bandelettes de test (112) afin de prélever une ou plusieurs bandelettes de test (118), un dispositif de comptage (134) pour le comptage d'un nombre et/ou d'une durée temporelle d'opérations d'ouverture du dispositif de fermeture (122), un support de données (136) pour le stockage de données spécifiques de charges des bandelettes de test (118) et/ou d'un nombre et/ou d'une durée temporelle d'opérations d'ouverture, un dispositif de transfert de données (144) pour le transfert sans fil de données spécifiques de charges et d'un nombre et/ou d'une durée temporelle d'opérations d'ouverture à un module d'analyse (114), dans un système de mesure (110) pour la mesure de concentrations de substances dans des milieux liquides.

9. Procédé pour la mesure de concentrations de substances dans des milieux liquides au moyen d'un système de mesure (110) selon l'une quelconque des revendications précédentes relatives à un système de mesure (110), **caractérisé par** les étapes suivantes:
a) on détecte un nombre et/ou une durée temporelle d'opérations d'ouverture au moyen du dispositif de comptage (134) et on le(s) stocke sur le support de données (136);
b) on transmet sans fil au module d'analyse (114) des données spécifiques de charges et un nombre et/ou une durée temporelle d'opérations d'ouverture; et
c) on détermine une concentration de substance dans un milieu liquide au moyen d'une bandelette de test (118).

10. Procédé selon la revendication précédente, avec l'étape supplémentaire suivante:
d) l'envoi d'un avertissement à un opérateur par le biais du module d'analyse (114) en cas de constatation d'un dépassement d'un nombre maximal d'opérations d'ouverture et/ou d'une durée temporelle totale maximale d'opérations d'ouverture du réservoir de bandelettes de test (112).
